# EUROPEAN PATENT SPECIFICATION

(11) **EP 3 909 621 B1**
(45) Date of publication and mention of the grant of the patent: **02.04.2025**
(21) Application number: 20707487.3
(22) Date of filing: 03.01.2020
(51) Int. Cl.: A61M 1/36, A61J 1/05, A61J 1/14, A61L 2/00

(54) **RECIPIENT FOR EX-VIVO TREATMENT OF BIOLOGICAL LIQUIDS**
BEHÄLTER FÜR EX-VIVO BEHANDLUNG VON BIOLOGISCHEN FLÜSSIGKEITEN
RÉCIPIENT POUR LE TRAITEMENT EX-VIVO DES FLUIDES BIOLOGIQUES

(30) Priority: 10.01.2019 ES 201930031 U
(43) Date of publication of application: 17.11.2021
(73) Proprietor: Meta Cell Technology SL, 08172 Sant Cugat (ES)
(72) Inventor: PINTO, Hernán, 08172 Sant Cugat (ES); PLAN, Philippe, 08172 Sant Cugat (ES)
(74) Representative: Curell Suñol S.L.P.
(86) International application number: PCT/ES2020/070004
(87) International publication number: WO 2020/144387

(56) References cited:
- US-A- 2 006 027
- US-B2- 8 296 071

## Description

### Field of the Invention

The invention is comprised in the field of devices for the activation of biomaterials, a concept which is also known as biostimulation.

More specifically, the invention relates to a container for the *ex vivo* treatment with electromagnetic radiation of biological fluids, in particular blood or other fluids such as plasma, sera, purified fluids, cell solutions, etc.

### State of the Art

There is a growing interest in the possibility of treatments by means of biostimulation. Direct treatments on the patient, for example, by irradiating a certain part of the patient's body with electromagnetic radiation of specific wavelengths, are known: the absorption of this light by a part of the tissue activates a photochemical reaction that may have potential therapeutic effect. The cell processes involved, as well as their potential medical or cosmetic applications, such as anti-inflammatory, analgesic, healing effects, etc., are still in the study phase.

Given the limitation involved in external use, the testing of techniques of this type in *ex vivo* techniques for biological fluids have also begun: for example, a blood sample is obtained from the patient, treated by means of biostimulation, and re-injected to obtain the therapeutic effect. Generally, the person performing the treatment extracts blood by means of a syringe and introduces the syringe itself into a device intended for biostimulation, for example, by means of electromagnetic irradiation. Once the irradiation ended, the blood is re-injected into the patient.

This type of method has various problems. On the one hand, handling syringes in this manner entails risks, given that they are in a form that is rather unsuitable for being transported and handled once they are full, so they may fall or become contaminated relatively easily. On the other hand, the many shapes and sizes of the syringes means that biostimulation is rather imprecise and subject to variations which may affect the end result.

A type of solution which allows solving the drawbacks set forth above, assuring, as much as possible, a safe, efficient, and precise method is required.

The following documents have been found to be relevant prior art for the subject-matter of the application:
- US 2 006 027 A (SAMUEL MOORE)
- US 8 296 071 B2 (EDRICH RICHARD ALAN [us]; GOODRICH LAURA [us] ET AL.)

Both documents describe examples of containers for *ex vivo* treatment of biological fluids which are permeable to electromagnetic radiation.

### Description of the Invention

Unless otherwise indicated, the expressions indicating coordinates, such as "above", "below", "left", "right", "upper", "lower", "front," or "rear" are used, for the sake of clarity, only by way of identification labels referring to the common way of usage of the container, in accordance with the attached drawings. The skilled person will understand that other coordinate systems are possible.

The purpose of the invention is to provide a container for the *ex vivo* treatment of biological fluids of the type indicated above, which allows solving the technical problems being considered above.

This purpose is achieved by means of a container of the type indicated above, having the technical features of claim 1.

Given that the closure means hermetically close the reservoir against gases and liquids, the skilled person will understand that the reservoir is closed except for said first port and second port, and that the material or materials used in the walls of the said reservoir do not allow the passage of gases or liquids. Furthermore, the use of the word *window* does not entail any type of opening. In fact, it is a region of the wall with a material and structure that is transparent to the passage of electromagnetic radiation in the working wavelength or in the range of working wavelengths. At the same time, since said window region is part of the wall of the reservoir, it must be rigid and prevents the passage of both liquids and gases. The container will be understood herein as being arranged in a closed position when said first closure means close said first access port and said second closure means close said second access port. The container will be understood that as being arranged in a half-open, half-closed, partially open, or partially closed position when only one of said first closure means or said second closure means are closing their corresponding access port. Finally, the container will be understood as being arranged in an open position when none of said closure means is closing their corresponding access port.

The rigidity of the container facilitates its handling and storage. It also provides structural strength. In turn, the fact that the reservoir is formed by rigid walls prevents its deformation and the subsequent movement of the sample therein, so it improves treatment predictability and uniformity, thereby being more precise. The container allows hermetic closure even against gases, which prevents possible contaminations in the sample to be treated. It is therefore possible to open the container, inject the sample, close the container to perform biostimulation treatment, and open it again to remove the treated sample.

On the other hand, the fact that it comprises two access ports allows, with the container in the open position, injecting the sample through one of said ports and leaving the air or gas that was previously in the reservoir to be displaced by the sample and exit through the other port. This minimizes production cost and prolongs the storage life of the container, given that it is not necessary to create a prior vacuum in the reservoir. Alternatively, in contrast to the case of containers with a single access port in which there is no prior vacuum, for the container of the invention it is not necessary for the air or gas to exit through the same port through which the sample is injected, which may cause the sample itself to splash out. This last case is particularly problematic due to the biological origin of the sample, for example in the case of blood, and the possible risk of contamination of the external environment or of the person performing the treatment. Similarly, when the treatment ends, removing the sample with the container open allows the sample to exit through one port and whereas air which facilitates sample extraction enters through the other port.

Preferred embodiments the features of which are described in the dependent claims have been provided based on the invention defined in the main claim.

Preferably, said electromagnetic radiation is comprised between infrared and ultraviolet radiation. Some of the treatments described in the art point to possible therapeutic effects in those bands of the electromagnetic spectrum, preferably between near infrared and the visible light spectrum, these being the bands in which greater applicability of biostimulation treatments is expected. For example, it has been described in the art that the irradiation of blood with low-intensity laser light of 660 nm (red light) significantly increases lymphocyte proliferation. When lymphocytes are irradiated in the presence of erythrocytes, an increase in the production of free radicals and lipid peroxides is also observed. Likewise, these ranges facilitate the selection of available materials.

In an advantageous embodiment, said inner thickness of said reservoir is equal to or less than 5 mm. This maximum thickness allows an effective and uniform sample irradiation. A greater thickness may imply that a large amount of energy may be required for some regions of the electromagnetic spectrum, in order to assure sufficient penetration. As a result, the layer closest to the radiation source may receive an energy level that would be outside the required margins of treatment. Generally, the smaller the thickness is, the more efficient and uniform the irradiation can be.

Preferably, said inner thickness of said reservoir is equal to or greater than 1 mm. The minimum thickness therefore allows biological fluids such as blood or plasma to flow smoothly inside the reservoir. Generally, the greater the thickness is, the more readily the sample can flow inside the reservoir.

Preferably, said inner thickness of said reservoir is comprised in the range of 1 to 4 mm, which entails a compromise between sample fluidity and irradiation efficiency and uniformity. Preferably, said inner thickness of said reservoir is 2,5 mm, this being an optimal intermediate point for fluids such as blood and irradiations in the visible, infrared, or ultraviolet region.

The inner volume of the reservoir is conditioned by its thickness and the dimensions of the container are necessarily affected by the dimensions of the reservoir. For this reason, the preceding ranges also allow manufacturing containers that can be easily handled for common sample volumes in biostimulation treatments.

Preferably, said reservoir has an inner volume between 2 and 30 ml. Generally, the fluid samples, and in particular, blood samples, are extracted from the body of the patient using a syringe. The preceding interval coincides with the volumetric capacities of common syringes. Advantageously, the fact that the reservoir of the container has a volumetric capacity coinciding with the syringe to be used allows the reservoir to be filled with a single extraction, reducing the possibility of sample contamination, for example, due to the container being left open during successive injections. At the same time, a completely full reservoir is achieved during treatment, which assures that said treatment is precise as the amount and volume of the sample, as well as the distribution thereof inside the reservoir, are predictable. Preferably, said inner volume is 10 ml, coinciding with the common syringes for blood extraction. The skilled person will understand that the volume of the reservoir herein defined refers to the volume contained between the walls forming the reservoir, without taking into account the possible conduits of the access ports, if said conduits exist. Therefore, the total inner volume of the container may be slightly greater than that of the reservoir, which is a preferred option, given that it reduces the possibility of the injected sample coming out through any of said access ports in an uncontrolled manner.

Preferably, said window region extends throughout the entire said upper wall or said lower wall having said window region. In other words, the window region entirely covers the upper wall or the lower wall in which said window region is provided, which implies maximum irradiation efficiency. The skilled person will understand that, due to manufacturing reasons, there may be a narrow frame which keeps the structure of the container together.

Preferably, both said upper wall and said lower wall each has a window region configured to allow the passage of electromagnetic radiation from the outside of said body to said reservoir. In other words, the container allows irradiation through both the upper wall and the lower wall. This is particularly advantageous as it allows flexibility in the design of the device performing the irradiation. Furthermore, it can maximize irradiation or even facilitate irradiating with different electromagnetic radiation sources or with electromagnetic radiation sources from different regions of the spectrum.

Preferably, said body is made of a polymeric material, simplifying the manufacture of the container and reducing costs associated therewith, for example using injection methods. Furthermore, materials of this type have a reduced weight in comparison, for example, to glass or crystal, and also allow increasing mechanical resistance to impacts, which is particularly advantageous in the case of containers containing sensitive or potentially contaminating samples that must be handled manually.

Preferably, said side wall, said upper wall, and said lower wall are formed in said body, resulting in a simple structure, which reduces costs and at the same time allows increasing robustness and general strength.

Preferably, said body comprises an upper part in which said upper wall is formed, and a lower part in which said lower wall is formed; said upper part and said lower part being attached in ribs forming said side wall of said reservoir. Preferably, the attachment between said ribs is done by means of ultrasonic welding. The configuration in only two parts has several advantages: it reduces production costs and simplifies the structure, especially for the case of polymer injection manufacturing. This simplicity also makes it more robust given that there are fewer parts to be assembled. On the other hand, in comparison to manufacturing in a single part, when made in separate parts, the manufacturing does not require internal machining for the reservoir, which minimizes manufacturing cost.

Preferably, said body is flat, and said upper wall and said lower wall are flat and parallel to one another. The flat shape facilitates storage and handling, in particular for use with the device or machine performing the biostimulation treatment. It also improves safety by reducing the risk of accidental falls during handling, for example, the risk of it being able to roll and fall when placed on a table.

Preferably, said body is rectangular, which facilitates handling and gripping by hand, as well as storage, especially when several containers are stored together. The flat rectangular cartridge-like shape also facilitates insertion into the device which performs the treatment and simplifies the design of the part where the container is deposited or inserted in said device. Preferably, said reservoir is rectangular.

In an advantageous embodiment, said container further comprises a casing made of elastomer, which improves the strength of the container, for example in the event of falls. The casing also improves gripping, thereby increasing safety during handling. Furthermore, it is an element that can be easily manufactured in different colors. This allows the containers to be readily identifiable and distinguishable from one another. This construction based on a body and a casing simplifies container manufacturing and allows greater flexibility in the manufacturing of each of its elements, reducing cost at the same time. Preferably, said elastomer is a medical grade silicone which assures that there will be no allergic reactions or reactions of another type even in the cases of direct contact or contamination, and which is also resistant to bacterial growth.

Preferably, said first closure means and said second closure means are formed in said casing. The reservoir is therefore closed by means of the casing itself. This simplifies the manufacture of the main body, for example, it allows the body to be entirely rigid and to have access ports, whereas the casing will have the closure means. Preferably, said first closure means and said second closure means comprise removable plugs formed in said casing. The design of the casing itself is therefore simplified and its handling facilitated.

Preferably, said body further comprises an identification region configured for receiving a label, said identification region not overlapping said window region. This element allows quick identification of the sample to be treated without interfering with the irradiation method for the biostimulation treatment. It reduces the probability of errors during the process, given that the identification can be directly incorporated to the container.

Preferably, said identification region is provided in the upper portion of said body, such that it is visible when the container is placed on a surface such as a table. Preferably, said identification region is provided close to one of the ends of said body. This facilitates access to the label and even allows holding the container by the end without affecting the window region or regions. This type of holding is useful in case the container must be introduced in a device or machine to perform the treatment.

Preferably, said first access port and said second access port each comprises a tube extending from said body and forming an access opening communicating the outside of said body with said reservoir. Therefore, the use of syringes for injecting the sample into the container is facilitated, which is particularly advantageous if the sample has been extracted from the patient by means of said syringe.

Preferably, the end of each of said tubes is provided with engagement means configured for taking in and fixing a syringe. Many of the syringes used in the market has a standardized end corresponding with engagement means that are also standardized, so one skilled in the art will have no problems in determining the type of engagement means depending on the syringe. The use of connections of this type increases safety by preventing the sample from spilling out when it is injected into the reservoir.

Preferably, said tubes are provided in the upper portion of said body, such that they do not cause instability if the container is left on a surface. Preferably, said tubes are provided close to one of the ends, thereby facilitating the complementary design in the device or machine which must receive said container to perform the biostimulation treatment of the sample.

In an advantageous embodiment, said container further comprises a sealed packaging containing said body and, where appropriate, said casing, in which said container is sterilized. Therefore, it is possible to assure safety and minimize the possibility of sample contamination. The assembly is packaged and sterilized, ready to be opened and used by the user in the moment when the treatment is to be performed.

Preferably, there is provided inside the reservoir a gas or a mixture of inert gases which favors the conservation of the container before use.

The invention also covers other detail features illustrated in the detailed description of an embodiment of the invention and in the attached drawings. The scope of the invention is defined by the appended claims.

### Brief Description of the Drawings

Figure 1 corresponds to a top view of an embodiment of the container of the invention. The window region 7 has been shaded to distinguish it from the upper wall. For the sake of clarity, this shading is not included in the rest of the drawings.
Figures 2A and 2B are perspective views of another embodiment of the container. In Figure 2A, the closure means are closed, whereas in Figure 2B they are open.
Figure 3 is a perspective view of the rigid body of the container according to the preceding embodiment.
Figures 4A and 4B are perspective views of the upper part of the body according to an embodiment of the invention. Figure 4A shows the top perspective view, whereas Figure 4B shows the same part from below.
Figure 5 is a perspective view of the lower part of the body according to the same embodiment as in Figures 4A and 4B.
Figure 6 is a perspective view of the casing for an embodiment of the container of the invention.
Figure 7 is a horizontal section of the body according to the embodiment of Figures 4A, 4B, and 5 in top view.
Figure 8 is a side view vertical section of the body according to the same embodiment of Figure 7.

### Detailed Description of Several Embodiments of the Invention

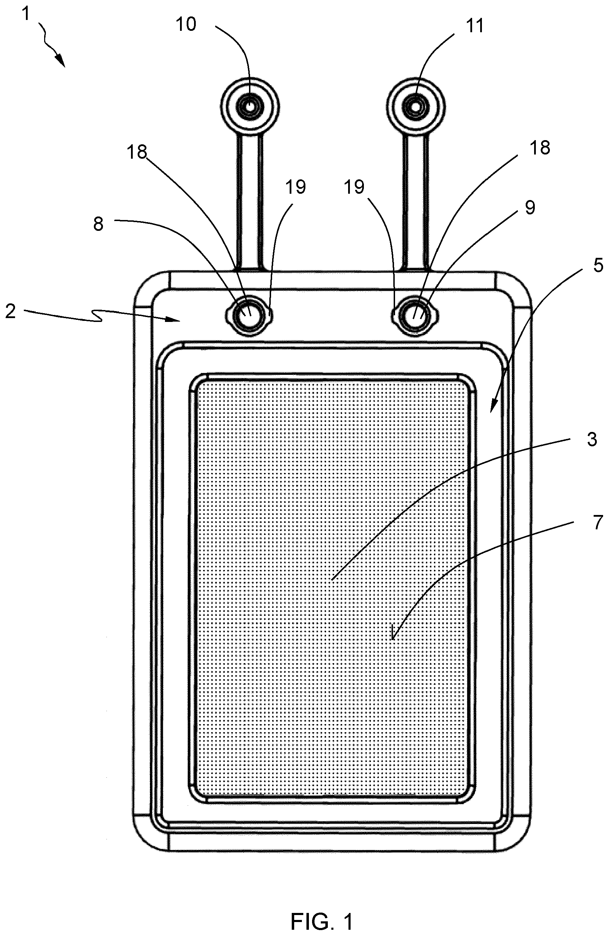

Figure 1 shows an embodiment of a container 1 for the *ex vivo* treatment of biological fluids, in particular blood. Said container 1 comprises a rigid body 2 which, in the example, is flat, rectangular, and made of a polymeric material. In particular, in the embodiment said polymeric material is an injection grade polymer based on methylmethacrylate acrylonitrile butadiene styrene (MABS) marketed by Terlux^{®} HD 2812.

A reservoir 3 is provided inside said body 2. Said reservoir is 3 formed by a side wall 4, a rigid upper wall 5, and a rigid lower wall 6. For the embodiments of the drawings, said upper wall 5 and said lower wall 6 are flat and parallel to one another. Said reservoir 3 has an inner thickness defined by the separation between said upper wall 5 and said lower wall 6. For the embodiments in which the container is intended for the *ex vivo* treatment of blood, said inner thickness is 2.5 mm.

At least one of said upper wall 5 or said lower wall 6 has a window region 7 configured to allow the passage of electromagnetic radiation from the outside of said body 2 to said reservoir 3. In the example of Figure 1, said window region 7 is present in said upper wall 5 and, for the sake of clarity, it has been shaded with cross hatching so that it stands out from of the rest of container. This shading does not appear in the rest of drawings.

For the embodiments herein described, said electromagnetic radiation is comprised between near infrared and the visible light spectrum. Other embodiments contemplate other ranges of the electromagnetic spectrum, such as ultraviolet radiation or even higher frequencies such as X-rays or lower frequencies in the range of the microwaves. Sample irradiation uniformity is affected by the penetration capacity of the radiation in said sample, and this in turn may depend on the band of the electromagnetic spectrum. For this reason, the skilled person will understand that the reservoir thickness limit of 5 mm mentioned above in this document may vary for some embodiments.

As can be seen in the examples of the drawings of the embodiments, said reservoir 3 has a rectangular shape. In particular, for the examples herein described in which the fluid to be treated is blood, reservoir 3 measures 74.4 x 54.4 mm. Given that the inner thickness is 2.5 mm, these dimensions correspond to a total volume of about 10 ml.

Said container 1 also comprises a first access port 8 for fluids to said reservoir 3 from the outside of said body 2, and a second access port 9 for fluids to said reservoir 3 from the outside of said body 2. For the examples of the drawings, said first access port 8 and said second access port 9 each comprises a tube 17 extending from said body 2 and forming an access opening 18 communicating the outside of said body 2 with said reservoir 3. Said tubes 17 are provided in the upper portion of said body 2 at one of the ends thereof. Additionally, in the examples of the drawings the end of each of said tubes 17 is provided with engagement means 19 configured for taking in and fixing a syringe.

On the other hand, the container 1 of Figure 1 also comprises first closure means 10 for said first port 8 and second closure means 11 for said second port 9. Both closure means 10 and 11 comprise removable plugs configured for being inserted into their corresponding openings 18 of the ports 8 and 9, and jointly providing a gas and liquid tight seal for said reservoir 3.

Other embodiments of the container 1 according to the invention sharing a large part of the features described in the preceding paragraphs are shown below. Accordingly, only the differentiating elements will be described hereinafter, whereas reference is made to the description of the first embodiment for common elements.

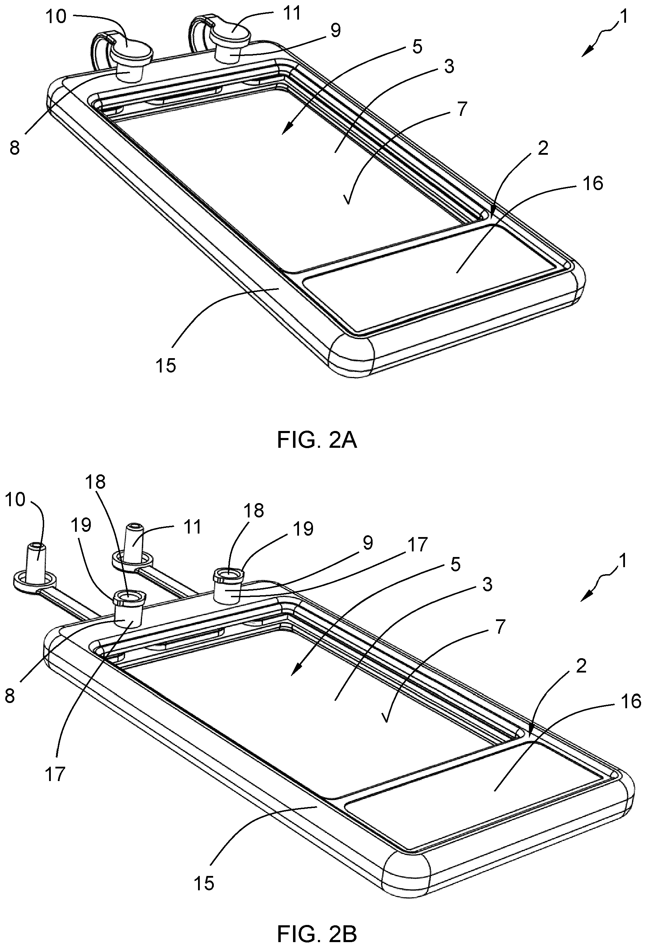

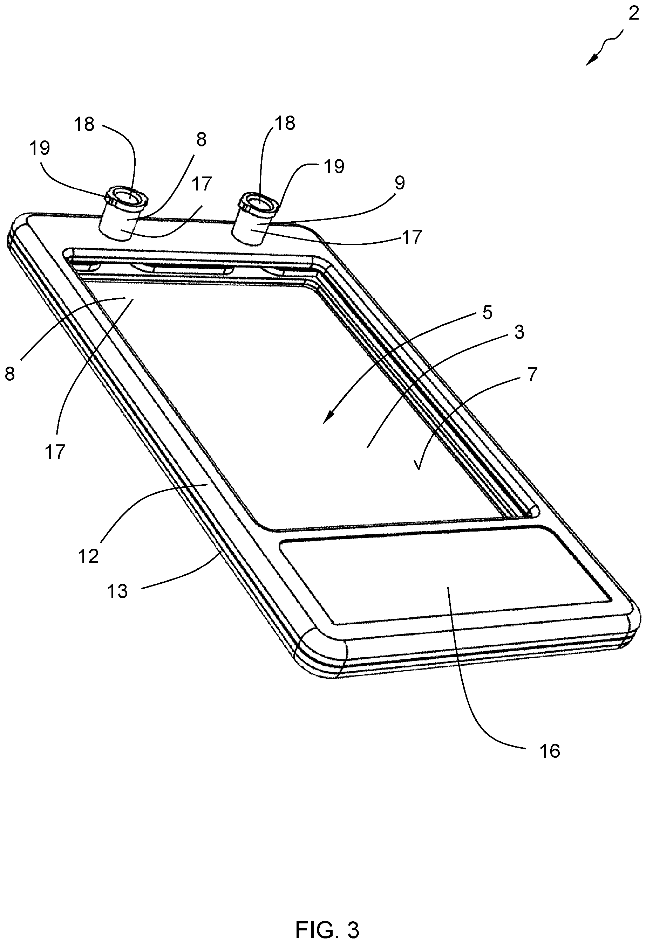

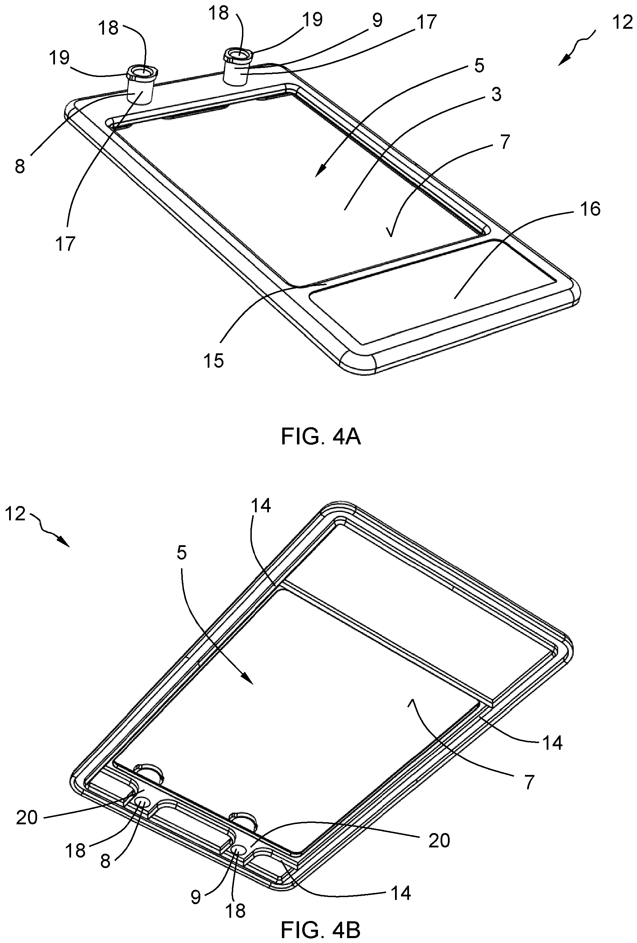

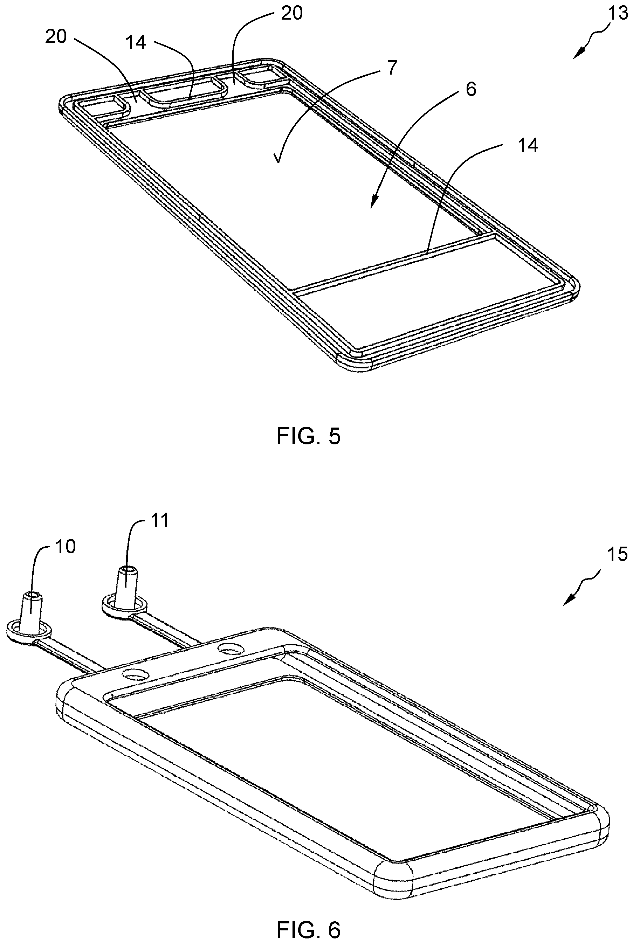

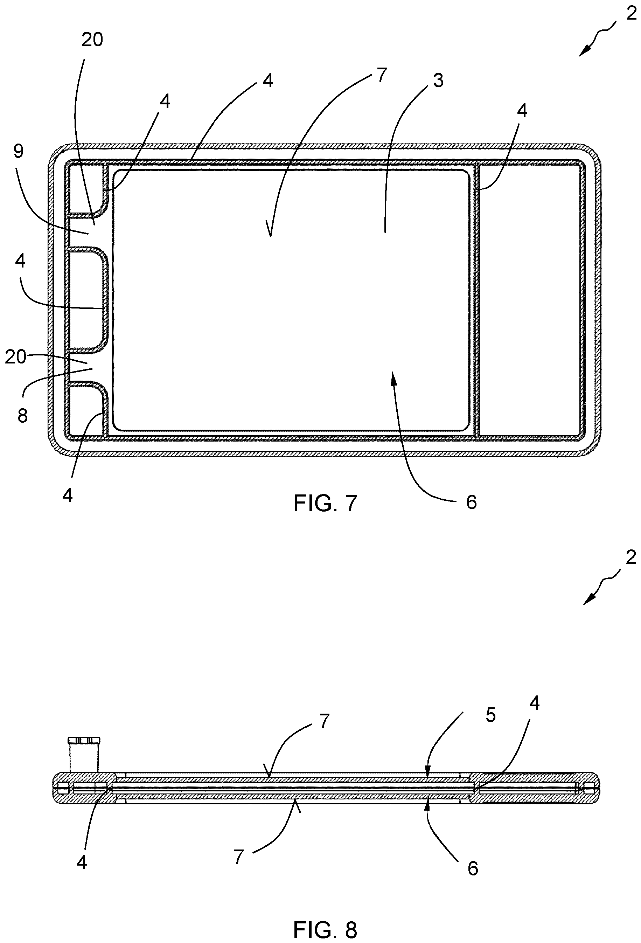

Figures 2 to 8 show another embodiment of the container 1 in which said side wall 4, said upper wall 5, and said lower wall 6 are formed in said body 2. In particular, in Figures 3, 4, and 5 it can be observed that said body 2 comprises an upper part 12 in which said upper wall 5 is formed, and a lower part 13 in which said lower wall 6 is formed. Said upper part 12 and said lower part 13 are attached in ribs 14 forming said side wall 4 of said reservoir 3. The upper part 12 is depicted separately in Figures 4A and 4B, whereas the lower part 13 is depicted separately in Figure 5. The body 2 resulting from the attachment of both parts 12 and 13 is shown in a perspective view in Figure 3, and by means of section views in Figures 7 and 8. To manufacture the container 1 of the example, the attachment between said ribs 14 is done by means of ultrasonic welding.

It can be observed in Figures 4A and 4B how the access ports 8 and 9 each comprises a tube 17 with an opening 18 going through the upper wall 5 and communicating with channels 20 that in turn connect with the inside of said reservoir 3. Said channels 20 can also be clearly observed in Figure 5 and Figure 7.

For the embodiment, both the upper wall 5 and the lower wall 6 each has a window region 7. In turn, these window regions 7 extend almost entirely throughout each respective wall 5 and 6. For manufacturing reasons, a small margin may actually exist between the side wall 4 and the window region 7. This can be seen in the cross-section view shown in Figure 7. Therefore, when "almost entirely" is used herein, it will be understood that there will be no more than 10% difference between the surface of the window region 7 and the surface of its corresponding wall 8 or 9.

On the other hand, the container 1 of this embodiment further comprises an identification region 16 configured for receiving a label, said identification region 16 not overlapping said window regions 7. During the manufacturing of the body 2, a textured region is created on the entire surface of said body 2, except in the window regions 7, and optionally also except in the identification region 16. Said textured region improves gripping and allows differentiating and providing different characteristics to the different regions of the body 2, even if the latter is manufactured in a single material, like in the case of the example.

As can be observed in the drawings, said identification region 16 is provided in the upper portion of said body 2, close to one of the ends of said body 2, in particular at the end opposite where the access ports 8 and 9 to the reservoir 3 are located.

In the example shown in Figures 2A, 2B, and 6, it can be seen how the container 1 further comprises a casing 15 made of elastomer which, for this example, is a medical grade silicone. The skilled person will understand that the definition of medical grade silicone depends on the particular regulations of each territory, and that these regulations may also change over time. In this example, both closure means 10 and 11 are formed in said casing 15 in the form of removable plugs.

In other embodiments, the container is intended for other biological fluids with a higher viscosity than blood, so the inner thickness of said reservoir 3 is greater than 2.5 mm, but less than 5 mm, in the case of using the same regions of the electromagnetic spectrum.

Still in other embodiments, the container is intended for other biological fluids with a lower viscosity than blood, so the inner thickness of said reservoir is less than 2.5 mm, where it can even be less than 1 mm for fluids of very low viscosity.

For some embodiments, said container 1 further comprises a sealed packaging containing said body 2 and in which said container 1 is sterilized.

## Claims

1. Container (1) for the *ex vivo* treatment of biological fluids, in particular blood, comprising:
- a rigid body (2);
- a reservoir (3) provided inside said body (2);
- formed by a side wall (4), a rigid upper wall (5), and a rigid lower wall (6);
- said reservoir (3) having an inner thickness defined by the separation between said upper wall (5) and said lower wall (6);
- at least one of said upper wall (5) or said lower wall (6) having a window region (7) configured to allow the passage of electromagnetic radiation from the outside of said body (2) to said reservoir (3);
- a first access port (8) for fluids to said reservoir (3) from the outside of said body (2);
- first closure means (10) for said first port (8);
- a second access port (9) for fluids to said reservoir (3) from the outside of said body (2);
- second closure means (11) for said second port (9);
said reservoir (3) being closed except for said first port (8) and second port (9);
**characterized in that** said first closure means (10) and said second closure means (11) being configured to jointly provide a gas and liquid tight seal for said reservoir (3).

2. Container (1) according to claim 1, **characterized in that** said inner thickness of said reservoir (3) is equal to or less than 5 mm.

3. Container (1) according to any of claims 1 or 2, **characterized in that** said inner thickness of said reservoir (3) is equal to or greater than 1 mm.

4. Container (1) according to any of claims 1 to 3, **characterized in that** said inner thickness of said reservoir (3) is comprised in the range of 1 to 4 mm, preferably being 2.5 mm.

5. Container (1) according to any of claims 1 to 4, **characterized in that** said reservoir (3) has an inner volume between 2 and 30 ml, preferably, 10 ml.

6. Container (1) according to any of claims 1 to 5, **characterized in that** said window region (7) extends throughout the entire said upper wall (5) or said lower wall (6) having said window region (7).

7. Container (1) according to any of claims 1 to 6, **characterized in that** both said upper wall (5) and said lower wall (6) each has a window region (7) configured to allow the passage of electromagnetic radiation from the outside of said body (2) to said reservoir (3).

8. Container (1) according to any of claims 1 to 7, **characterized in that** said electromagnetic radiation is comprised between infrared and ultraviolet radiation, preferably, between near infrared and the visible light spectrum.

9. Container (1) according to any of claims 1 to 8, **characterized in that** said body (2) is made of a polymeric material.

10. Container (1) according to any of claims 1 to 9, **characterized in that** said side wall (4), said upper wall (5), and said lower wall (6) are formed in said body (2), preferably, wherein said body (2) comprises an upper part (12) in which said upper wall (5) is formed, and a lower part (13) in which said lower wall (6) is formed; said upper part (12) and said lower part (13) being attached in ribs (14) forming said side wall (4) of said reservoir (3), more preferably by means of ultrasonic welding.

11. Container (1) according to any of claims 1 to 10, **characterized in that** said body (2) is flat, preferably rectangular, and said upper wall (5) and said lower wall (6) are flat and parallel to one another, wherein said reservoir is preferably rectangular.

12. Container (1) according to any of claims 1 to 11, **characterized in that** it further comprises a casing (15) made of elastomer, preferably medical grade silicone, wherein said first closure means (10) and said second closure means (11) are preferably formed in said casing (15), more preferably in the form of removable plugs.

13. Container (1) according to any of claims 1 to 12, **characterized in that** said body (2) further comprises an identification region (16), preferably provided in the upper portion of said body (2), more preferably close to one of the ends of said body (2), and configured for receiving a label, said identification region (16) not overlapping said window region (7).

14. Container (1) according to any of claims 1 to 13, **characterized in that** said first access port (8) and said second access port (9) each comprises a tube (17) extending from said body (2) and forming an access opening (18) communicating the outside of said body (2) with said reservoir (3), wherein said tubes (17) are preferably provided in the upper portion of said body (2), more preferably close to one of the ends, and wherein the end of each of said tubes (17) is preferably provided with engagement means (19) configured for taking in and fixing a syringe.

15. Container (1) according to any of claims 1 to 14, **characterized in that** it further comprises a sealed packaging containing said body (2) and, in which said container (1) is sterilized.

## Patentansprüche

1. Behälter (1) für ex-vivo-Behandlung von biologischen Flüssigkeiten, insbesondere von Blut, umfassend:
- einen starrer Körper (2);
- ein Reservoir (3), das im Inneren des Körpers (2) vorgesehen ist;
- gebildet durch eine Seitenwand (4), eine starre obere Wand (5) und eine starre untere Wand (6),
- wobei der Behälter (3) eine innere Dicke aufweist, die durch den Abstand zwischen der oberen Wand (5) und der unteren Wand (6) definiert ist;
- wobei mindestens eine der oberen Wand (5) oder der unteren Wand (6) einen Fensterbereich (7) aufweist, der dazu eingerichtet ist, einen Durchgang von elektromagnetischer Strahlung von der Außenseite des Körpers (2) zu dem Behälter (3) zu ermöglichen;
- eine erste Zugangsöffnung (8) für Fluide zu dem Reservoir (3) von der Außenseite des Körpers (2);
- ein erstes Verschlussmittel (10) für die erste Öffnung (8);
- eine zweite Zugangsöffnung (9) für Fluide zu dem Reservoir (3) von der Außenseite des Körpers (2);
- ein zweites Verschlussmittel (11) für die zweite Öffnung (9);
wobei das Reservoir (3) mit Ausnahme der ersten (8) und zweiten (9) Öffnung geschlossen ist;
**dadurch gekennzeichnet, dass** das erste Verschlussmittel (10) und das zweite Verschlussmittel (11) dazu eingerichtet sind, gemeinsam eine gas- und flüssigkeitsdichte Abdichtung für das Reservoir (3) bereitzustellen.

2. Behälter (1) nach Anspruch 1, **dadurch gekennzeichnet, dass** die innere Dicke des Reservoirs (3) gleich oder kleiner als 5 mm ist.

3. Behälter (1) nach einem der Ansprüche 1 oder 2, **dadurch gekennzeichnet, dass** die innere Dicke des Reservoirs (3) gleich oder größer als 1 mm ist.

4. Behälter (1) nach einem der Ansprüche 1 bis 3, **dadurch gekennzeichnet, dass** die innere Dicke des Behälters (3) im Bereich von 1 bis 4 mm liegt, vorzugsweise bei 2.5 mm.

5. Behälter (1) nach einem der Ansprüche 1 bis 4, **dadurch gekennzeichnet, dass** das Reservoir (3) ein Innenvolumen zwischen 2 und 30 ml, vorzugsweise 10 ml, hat.

6. Behälter (1) nach einem der Ansprüche 1 bis 5, **dadurch gekennzeichnet, dass** der Fensterbereich (7) sich über die gesamte obere Wand (5) erstreckt oder die untere Wand (6) den Fensterbereich (7) hat.

7. Behälter (1) nach einem Ansprüche 1 bis 6, **dadurch gekennzeichnet, dass** sowohl die obere Wand (5) als auch die untere Wand (6) jeweils einen Fensterbereich (7) hat, der dazu eingerichtet ist, den Durchgang von elektromagnetischer Strahlung von der Außenseite des Körpers (2) zu dem Behälter (3) zu ermöglichen.

8. Behälter (1) einem Ansprüche 1 bis 7, **dadurch gekennzeichnet, dass** die elektromagnetische Strahlung zwischen Infrarot- und Ultraviolettstrahlung enthalten ist, vorzugsweise zwischen dem nahen Infrarot und dem sichtbaren Lichtspektrum.

9. Behälter (1) nach einem der Ansprüche 1 bis 8, **dadurch gekennzeichnet, dass** der Körper (2) aus einem polymeren Material hergestellt ist.

10. Behälter (1) nach einem der Ansprüche 1 bis 9, **dadurch gekennzeichnet, dass** die Seitenwand (4), die obere Wand (5) und die untere Wand (6) in dem Körper (2) ausgebildet sind, wobei der Körper (2) vorzugsweise einen oberen Teil (12), in dem die obere Wand (5) ausgebildet ist, und einen unteren Teil (13), in dem die untere Wand (6) ausgebildet ist, umfasst, wobei der oberen Teil (12) und der unteren Teil (13), durch Innenrippen (14) befestigt sind, welche die Seitenwand (4) des Behälters (3) bilden, vorzugsweise durch Ultraschallschweißen.

11. Behälter (1) nach einem der Ansprüche 1 bis 10, **dadurch gekennzeichnet, dass** der Körper (2) flach, vorzugsweise rechteckig, ist und die obere Wand (5) und die untere Wand (6) flach und parallel sind, wobei das Reservoir vorzugsweise rechteckig ist.

12. Behälter (1) nach einem der Ansprüche 1 bis 11, **dadurch gekennzeichnet, dass** er ferner ein Gehäuse (15) aus Elastomer, vorzugsweise medizinischem Silikon, aufweist, wobei das erste Verschlussmittel (10) und das zweite Verschlussmittel (11) vorzugsweise in dem Gehäuse (15) ausgebildet sind, besonders bevorzugt in Form von entfernbaren Stopfen.

13. Behälter (1) nach einem der Ansprüche 1 bis 12, **dadurch gekennzeichnet, dass** der Körper (2) ferner einen Identifikationsbereich (16) umfasst, der vorzugsweise im oberen Teil des Körpers (2), besonders bevorzugt in der Nähe eines der Enden des Körpers (2), vorgesehen und dazu eingerichtet ist, ein Etikett aufzunehmen, wobei der Identifikationsbereich (16) den Fensterbereich nicht überlappt.

14. Behälter (1) nach einem der Ansprüche 1 bis 13, **dadurch gekennzeichnet, dass** die erste Zugangsöffnung (8) und die zweite Zugangsöffnung (9) jeweils eine Röhre (17) umfassen, die sich von dem Körper (2) weg erstreckt und eine Zugangsöffnung (18) bildet, die eine Verbindung zwischen der Außenseite des Körpers (2) mit dem Reservoir (3) herstellt, wobei die Röhren (17) vorzugsweise im oberen Teil des Körpers (2), vorzugsweise in der Nähe eines der Enden, vorgesehen sind und wobei das Ende jedes der Röhren vorzugsweise mit Eingriffsmitteln (19) versehen ist, die für die Aufnahme und Befestigung einer Spritze eingerichtet sind.

15. Behälter (1) nach einem der Ansprüche 1 bis 14, **dadurch gekennzeichnet, dass** er außerdem eine versiegelte Verpackung umfasst, die den Körper (2) enthält und in welcher der Behälter (1) sterilisiert wird.

## Revendications

1. Récipient (1) pour le traitement *ex vivo* de fluides biologiques, notamment de sang, comprenant :
- un corps (2) rigide;
- un réservoir (3) disposé à l'intérieur dudit corps (2);
- formé par une paroi latérale (4), une paroi supérieure (5) rigide et une paroi inférieure (6) rigide;
- ledit réservoir (3) présentant une épaisseur intérieure définie par la séparation entre ladite paroi supérieure (5) et ladite paroi inférieure (6);
- au moins l'une de ladite paroi supérieure (5) ou de ladite paroi inférieure (6) présentant une région de fenêtre (7) configurée pour permettre le passage de rayonnement électromagnétique de l'extérieur dudit corps (2) audit réservoir (3) ;
- un premier orifice d'accès (8) pour les fluides audit réservoir (3) depuis l'extérieur dudit corps (2) ;
- des premiers moyens de fermeture (10) pour ledit premier orifice (8);
- un second orifice d'accès (9) pour les fluides audit réservoir (3) depuis l'extérieur dudit corps (2);
- des seconds moyens de fermeture (11) pour ledit second orifice (9);
ledit réservoir (3) étant fermé à l'exception dudit premier orifice (8) et dudit second orifice (9);
**caractérisé en ce que** lesdits premiers moyens de fermeture (10) et lesdits seconds moyens de fermeture (11) sont configurés pour assurer conjointement une étanchéité aux gaz et aux liquides dudit réservoir (3).

2. Récipient (1) selon la revendication 1, **caractérisé en ce que** ladite épaisseur intérieure dudit réservoir (3) est égale ou inférieure à 5 mm.

3. Récipient (1) selon l'une quelconque des revendications 1 ou 2, **caractérisé en ce que** ladite épaisseur intérieure dudit réservoir (3) est égale ou supérieure à 1 mm.

4. Récipient (1) selon l'une quelconque des revendications 1 à 3, **caractérisé en ce que** ladite épaisseur intérieure dudit réservoir (3) est comprise dans la plage de 1 à 4 mm, de préférence étant de 2.5 mm.

5. Récipient (1) selon l'une quelconque des revendications 1 à 4, **caractérisé en ce que** ledit réservoir (3) présente un volume intérieur compris entre 2 et 30 ml, de préférence 10 ml.

6. Récipient (1) selon l'une quelconque des revendications 1 à 5, **caractérisé en ce que** ladite région de fenêtre (7) s'étend sur toute ladite paroi supérieure (5) ou ladite paroi inférieure (6) présentant ladite région de fenêtre (7).

7. Récipient (1) selon l'une quelconque des revendications 1 à 6, **caractérisé en ce qu'**à la fois ladite paroi supérieure (5) et ladite paroi inférieure (6) présentent chacune une région de fenêtre (7) configurée pour permettre le passage de rayonnement électromagnétique de l'extérieur dudit corps (2) audit réservoir (3).

8. Récipient (1) selon l'une quelconque des revendications 1 à 7, **caractérisé en ce que** ledit rayonnement électromagnétique est compris entre le rayonnement infrarouge et le rayonnement ultraviolet, de préférence entre le spectre proche infrarouge et le spectre de lumière visible.

9. Récipient (1) selon l'une quelconque des revendications 1 à 8, **caractérisé en ce que** ledit corps (2) est en matériau polymère.

10. Récipient (1) selon l'une quelconque des revendications 1 à 9, **caractérisé en ce que** ladite paroi latérale (4), ladite paroi supérieure (5) et ladite paroi inférieure (6) sont formées dans ledit corps (2), de préférence, dans lequel ledit corps (2) comprend une partie supérieure (12) dans laquelle est formée ladite paroi supérieure (5), et une partie inférieure (13) dans laquelle est formée ladite paroi inférieure (6); ladite partie supérieure (12) et ladite partie inférieure (13) étant fixées dans des nervures (14) formant ladite paroi latérale (4) dudit réservoir (3), plus préférentiellement par soudage par ultrasons.

11. Récipient (1) selon l'une quelconque des revendications 1 à 10, **caractérisé en ce que** ledit corps (2) est plat, de préférence rectangulaire, et ladite paroi supérieure (5) et ladite paroi inférieure (6) sont plates et parallèles l'une à l'autre, dans lequel ledit réservoir est de préférence rectangulaire.

12. Récipient (1) selon l'une quelconque des revendications 1 à 11, **caractérisé en ce qu'**il comprend en outre un boîtier (15) en élastomère, de préférence en silicone de qualité médicale, dans lequel lesdits premiers moyens de fermeture (10) et lesdits seconds moyens de fermeture (11) sont de préférence formés dans ledit boîtier (15), plus préférentiellement sous forme de bouchons amovibles.

13. Récipient (1) selon l'une quelconque des revendications 1 à 12, **caractérisé en ce que** ledit corps (2) comprend en outre une région d'identification (16), de préférence disposée dans la partie supérieure dudit corps (2), plus préférentiellement proche de l'une des extrémités dudit corps (2), et configurée pour recevoir une étiquette, ladite région d'identification (16) ne chevauchant pas ladite région de fenêtre (7).

14. Récipient (1) selon l'une quelconque des revendications 1 à 13, **caractérisé en ce que** ledit premier orifice d'accès (8) et ledit second orifice d'accès (9) comprennent chacun un tube (17) s'étendant à partir dudit corps (2) et formant une ouverture d'accès (18) mettant en communication l'extérieur dudit corps (2) avec ledit réservoir (3), dans lequel lesdits tubes (17) sont de préférence disposés dans la partie supérieure dudit corps (2), plus préférentiellement proche de l'une des extrémités, et dans lequel l'extrémité de chacun desdits tubes (17) est de préférence munie de moyens de mise en prise (19) configurés pour accueillir et fixer une seringue.

15. Récipient (1) selon l'une quelconque des revendications 1 à 14, **caractérisé en ce qu'**il comprend en outre un emballage scellé contenant ledit corps (2) et, dans lequel ledit récipient (1) est stérilisé.
